(19) **Europäisches Patentamt / European Patent Office / Office européen des brevets**

(11) **EP 3 548 129 B1**

(12) **EUROPEAN PATENT SPECIFICATION**

(45) Date of publication and mention
of the grant of the patent:
**23.03.2022  Bulletin 2022/12**

(21) Application number: **17807886.1**

(22) Date of filing: **04.12.2017**

(51) International Patent Classification (IPC):
**A61M 16/04** (2006.01)     **A61M 16/08** (2006.01)

(52) Cooperative Patent Classification (CPC):
**A61M 16/04; A61M 16/0463; A61M 16/0841;**
A61M 16/01; A61M 16/1095; A61M 16/202;
A61M 2205/3368; A61M 2205/3653;
A61M 2230/437

(86) International application number:
**PCT/EP2017/081367**

(87) International publication number:
**WO 2018/104224 (14.06.2018 Gazette 2018/24)**

(54) **DEVICES FOR SAMPLING EXHALED AIR**

VORRICHTUNGEN ZUR PROBENAHME AUS GEATMETER LUFT

DISPOSITIFS D'ÉCHANTILLONNAGE DE L'AIR EXPIRÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **05.12.2016  EP 16382589**

(43) Date of publication of application:
**09.10.2019  Bulletin 2019/41**

(73) Proprietor: **Servei Central d'Anestesiologia SLP
08022 Barcelona (ES)**

(72) Inventors:
• **RODIERA OLIVÉ, José Javier
08022 Barcelona (ES)**
• **CALLICÓ ROS, Ferran
17003 Girona (ES)**

(74) Representative: **ZBM Patents - Zea, Barlocci &
Markvardsen
Rambla Catalunya, 123
08008 Barcelona (ES)**

(56) References cited:
EP-A2- 0 112 668       WO-A1-89/11245
WO-A1-99/29358        WO-A1-2011/014543
WO-A1-2015/186122    WO-A1-2017/182757
WO-A2-02/13885        WO-A2-2009/144731
GB-A- 1 528 279       GB-A- 2 033 759
US-A- 4 167 667       US-A- 4 705 543
US-A1- 2013 098 365   US-A1- 2014 238 398
US-A1- 2016 345 863   US-A9- 2011 087 123

Printed by Jouve, 75001 PARIS (FR)

**Description**

The present disclosure relates to sampling devices, in particular to sampling devices for sampling the exhaled air of a patient. The present disclosure further relates to methods and systems for sampling exhaled air of a patient. The present application claims the benefit and priority of European patent application n° 16 382 589.6, filed on December 5, 2016.

BACKGROUND

In some circumstances, artificial ventilation may be applied to patients who are unable to breathe on their own, for example during surgery with general anesthesia or in a coma.

Artificial ventilation may be carried out manually or by a mechanically controlled ventilator e.g. a pump. Regardless of the ventilation type, the breathing cycle comprises two main phases: an inspiration phase wherein a gas mixture e.g. air, is conveyed into the patient; and an exhalation phase wherein the gas expelled by the lungs is sucked to remove it from the patient.

When mechanical ventilation is used, the patient is usually intubated i.e. an endotracheal tube is placed into his/her oropharyngeal cavity by pushing it through the patient trachea. Such endotracheal tube is generally a flexible and/or curved tube adaptable to the patient internal cavity. The endotracheal tube comprises a ventilation passageway which permits the gas to reach the lungs.

The gas provided to the patient may be $O_2$, or it may be a mixture of air and other gases, e.g. anaesthetic drugs. The exhaled gas mixture usually comprises released $CO_2$ and other gases such as $O_2$ and other volatile components.

When a patient is subjected to surgery under general anesthesia, several medications, e.g. sedative or anaesthetic drugs, are administered e.g. intravenously. These anaesthetic drugs, e.g. propofol, are metabolized by the body in different degrees depending on the individual circumstances. A small quantity of the products is released e.g. by transpiration and/or breathing, and it is known that a correlation exists between the concentration of a certain administered medication in the exhaled air and the concentration in the blood of the patient.

It may therefore be convenient to measure the amount or proportion of certain compounds in the air exhaled by a patient undergoing artificial ventilation. For example, during general anesthesia this may assist the anaesthetist in controlling the patient condition.

Some of the exhaled compounds exist in high concentrations and/or have high volatility, i.e. the compound is in gaseous state at atmospheric pressure, and thus, are easily detected by conventional technology that involves sending to an analyser part of the exhaled air that is sucked through the endotracheal tube.

However, certain substances of the exhaled air composition are not easily detected by conventional technology. Some substances may have a reduced volatility, i.e. they are not in gaseous state at atmospheric pressure. Additionally, reduced concentrations e.g. in the range of ppb (parts per billion) or even ppt (parts per trillion), of such substances may be expelled e.g. through respiration. Furthermore, sometimes the electro-chemical properties of the substances cause a tendency of the particles to adhere e.g. to the inner wall of the endotracheal tube or other sampling tube, and therefore the concentrations received by the analyser are very small and do not correspond to the real concentrations in the exhaled air. Particles that adhere to a conduit wall during a first exhalation may later become detached from the wall, and may be wrongly counted in a subsequent exhalation.

Other sources of contamination of a sample of exhaled air may be water vapour that condenses on the inner wall of the endotracheal tube and/or body fluids from the patient, and/or the air provided to the patient through the endotracheal tube.

As a consequence, the analyses of some compounds in the exhaled air carried out with known methods and devices are not always accurate and reliable.

It is known to sample the exhaled air through a sampling device that is connected to the endotracheal tube. However, the sampling line of known sampling devices is connected to the connection hub between the endotracheal tube and the analyser.

GB2033759 shows a device comprising an endotracheal tube and a monitoring line mounted accurately and securely relative to a central axis of the endotracheal tube.

In conclusion, there is a need for an exhaled air sampling device and/or method which permits a high accuracy and which at least partially solves some of the aforementioned problems.

SUMMARY

In a first aspect of the present invention, a device for sampling exhaled air of a patient is provided. The device comprises an endotracheal tube and a sampling probe for sampling exhaled air of a patient. The endotracheal tube comprises a ventilation passageway, a proximal end to be connected to a connection hub and a distal end to be placed inside a patient oropharyngeal cavity. The sampling probe comprises a sampling conduit and is mounted within the endotracheal tube. The sampling probe comprises a proximal end adapted to be connected to a fluid analyser and a distal end adapted to receive samples of the exhaled air, such that the probe is adapted to convey the samples to the fluid analyser, and wherein such distal end is placed at the distal end of the endotracheal tube. The distal end of the sampling probe is arranged separate from the inner wall of the endotracheal tube, and wherein the internal diameter of the sampling conduit is equal to or lower than 3 mm.

Exhaled air sampling may include measuring the amount or proportion of certain compounds in the air exhaled by a patient undergoing artificial ventilation, for example, during anesthesia. In particular, measuring the amount of certain substances of the exhaled air composition that are not easily detected by conventional technology, such as substances with reduced volatility and/or reduced concentrations in exhaled air, e.g. in the range of ppbs or ppts, for example propofol and other anesthetic gases.

By maintaining the distal end of the sampling conduit separate from the inner wall of the endotracheal tube, i.e. at a distance from the wall, the condensation droplets that may form on this wall are substantially prevented from entering the conduit and reaching the analyser, and the contamination of the sampled air is substantially avoided or reduced.

According to an example, the internal diameter of the sampling conduit is between 0.5 and 3 mm, preferably between 1 and 2 mm.

In some examples the sampling probe, with the sampling conduit, may be kept separated from the inner wall of the endotracheal tube by a separating piece that is attached to the sampling probe. The separating piece may comprise flaps, for example two or more relatively flexible, substantially radial flaps that extend from the sampling probe such that their distal ends engage the inner wall of the endotracheal tube.

At least part of the sampling probe, for example at least the part that is not close to the ends thereof, may be arranged adjacent to the wall of the endotracheal tube. For example it may be arranged inside a hollow passageway formed in the endotracheal tube wall, or it may be attached to the endotracheal tube wall by any suitable method.

According to the present invention, the device further comprises a heating element to heat at least a portion of the sampling probe. By heating at least a portion of the sampling probe the substances to be sampled, for example particles of certain products such as propofol, are less likely to adhere to the inner wall of the sampling conduit of the probe so that the proportion of exhaled particles that reach the fluid analyser is at least increased and the accuracy of the measure is improved.

According to the present invention, the heating element is a resistor which is coiled around at least a portion of the sampling conduit, for example along the entire length of the sampling conduit. Using a coiled resistor is a safe and efficient way to heat the desired portions of the probe.

According to an example, at least an external insulating layer for covering the heating element is provided, e.g. made of an electrically and/or thermal insulating material.

According to an example, the sampling conduit is made of a material with low chemical reactivity to a compound to be analysed.

According to an example, the sampling conduit is made of a material that has a coefficient of friction equal to or lower than 0.35.

According to an example, the sampling conduit is made of a material selected among PEEK, modified PEEK, PET, PTFE and PPS.

According to an example, the device further comprises at least a temperature sensor positioned to measure the temperature of at least one portion of the sampling probe.

According to an example, a valve may be provided for selectively allowing exhaled air to enter the fluid analyser. Such a valve may be suitable for allowing only exhaled air to reach the fluid analyser, but not air conveyed to the patient through the endotracheal tube, and thus, the analyses would not be contaminated by the incoming air flow.

In a second aspect of the present invention, a system for analysing composition of exhaled air is provided. The system comprises a device according to any of the disclosed examples, a fluid analyser and a ventilator.

In a further aspect, not being part of the present invention, a method for analysing the composition of exhaled air is provided. Firstly a device according to any of the disclosed examples is placed into a patient oropharyngeal cavity, secondly the exhaled air is sampled at the distal end of the endotracheal tube, and then the exhaled air composition is analysed.

The present invention is defined by the appended claims.

BRIEF DESCRIPTION OF THE DRAWINGS

Non-limiting examples of the present disclosure will be described in the following, with reference to the appended drawings, in which:

Figure 1 schematically illustrates a side view of a medical device according to an example;

Figure 2 schematically illustrates a portion of a sampling probe, according to the present invention;

Figure 3 schematically illustrates an example of a sampling probe;

Figures 4a - 4c schematically illustrate examples of the distal end of an endotracheal tube comprising an integrated sampling probe;

Figure 5 schematically illustrates an exemplary separating piece;

Figure 6 schematically illustrates a system for analysing exhaled air of a patient, according to the present invention;

Figures 7a and 7b schematically illustrate a system for analysing the exhaled air of a patient, which comprises a valve;

Figure 8 schematically illustrates an example of a method for analysing exhaled air, that is not part of the present invention;

Figure 9 illustrates values of measured propofol for different internal diameters of a sampling conduit; and

Figure 10 illustrates values of instantaneous speed in laminar flow for different internal diameters of a sampling conduit.

## DETAILED DESCRIPTION

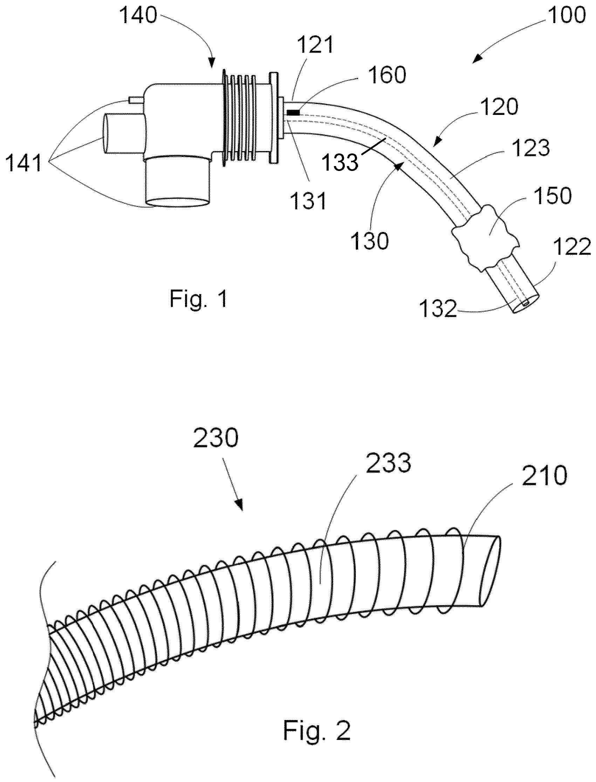

Figure 1 shows a medical device 100 for sampling exhaled air, according to implementations disclosed herein, which may be used in conditions of artificial ventilation in an anaesthetised patient.

In the example shown, the device 100 comprises a connection hub 140, an endotracheal tube 120 and a sampling probe 130 mounted within the endotracheal tube 120. The hub comprises a plurality of ports 141 for e.g. allowing sampling exhaled air and/or conveying air between a ventilator and a patient.

The endotracheal tube 120 comprises a ventilation passageway 123 to convey in and out of the lungs of the patient the air pumped from a ventilator (not shown), a proximal end 121 connected to the connection hub 140 and a distal end 122 to be placed inside a patient oropharyngeal cavity. The endotracheal tube in this example is made of e.g. Polyethylene (PE), Thermoplastic Polyurethane (TPU), silicone or similar material.

In the example of Figure 1, the endotracheal tube 120 may comprise an inflatable cuff 150 which, after being inflated, retains the endotracheal tube 120 inside the oropharyngeal cavity. The distal end of the endotracheal tube 120 is shown cut at right angle, but it may have any other suitable shape, as long as it enables intubating a patient, e.g. slope, etc. The tube 120 may also include other features such as a hole (not shown) to impede the occlusion of higher positioned bronchus.

The device 100 of the example of Figure 1 comprises a sampling probe 130 mounted within the endotracheal tube 120. The sampling probe 130 comprises a sampling conduit 133, a proximal end 131 connected via the hub 140 to a fluid analyser (not shown) e.g. a PTR-QMS 500 commercially available from IONICON, and a distal end 132 which is positioned in the vicinity of, i.e. at or near, the distal end of the endotracheal tube and is intended to take samples of the exhaled air, i.e. to receive part of the flow of exhaled air, and convey this air towards the fluid analyser.

The sampling conduit 133 in the example herein disclosed may be made of Polyether ether ketone (PEEK) as it has a low chemical reactivity regarding the substances to be sampled and analysed, for example propofol, and it is about 2 - 3 m long. However, the sampling conduit may also be made of modified PEEK, e.g. PEEK reinforced with Carbon fibres, Graphite and PTFE, Polyethylene terephthalate (PET), Polytetrafluoroethylene (PTFE), Polyphenylene sulfide (PPS) or similar materials with low chemical reactivity with respect to the substances to be sampled, e.g. to propofol, and may also have a coefficient of friction equal to or lower than 0.35. In addition, the sampling conduit 133 may have different lengths. The internal diameter of the sampling conduit may be between 0.5 and 3 mm, preferably between 1 and 2 mm.

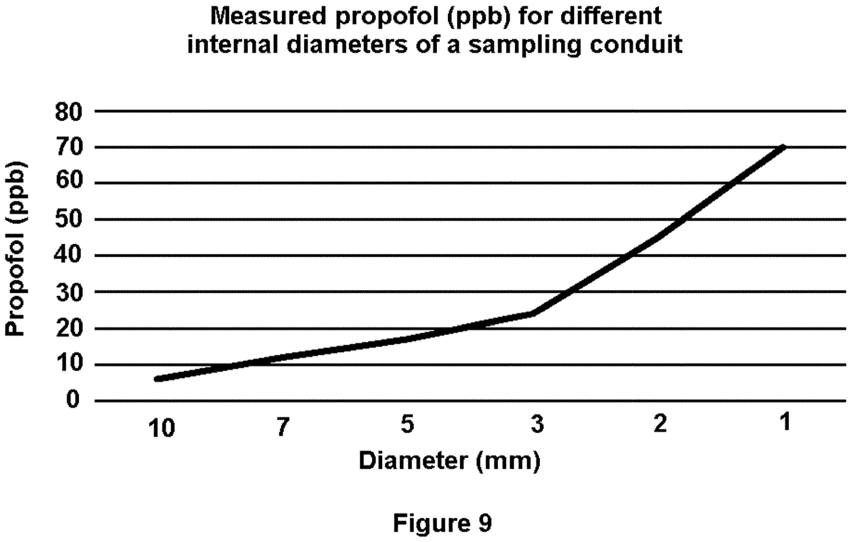

A study was carried out to evaluate the performance of several configurations of the device, and the results show a non-linear behaviour for different diameters of the sampling conduit. Figure 9 shows values of propofol concentration (in ppbs) measured i.e. the amount of propofol that reaches the analyser, in the exhaled air of anaesthetised patients using sampling conduits of different internal diameters. As depicted in the figure, it was found that the measured concentration of propofol increases for sampling conduits having diameters equal to or lower than 3 mm.

In the experiment, the anaesthetised patients were administered a propofol (vein) infusion having a concentration of 3 $\mu$g/ml and the exhaled air was pumped out under a flow rate of $5 \times 10^{-6}$ m$^3$/s and at a temperature of about 35° C. In addition, the analysed fluid, i.e. the exhaled air, had a cinematic viscosity ($v_c$) of about $1.66 \times 10^{-5}$ Pa.s and a density (d) of about 1.145 kg/m$^3$.

Firstly, the average speed ($V_m$) of the fluid was calculated for different inner diameters (D) by the following formula:

$$V_m = \frac{Q}{S}$$

Wherein Q is the predetermined suction flow of the ventilator i.e. $5 \times 10^{-6}$ m³/s, and S is the surface of the (circular) sampling conduit. The results obtained are shown in Table 1 below.

Table 1. Average speed of the fluid for different diameters.

| D (mm) | S (m²) | Q (m³/s) | $V_m$ (m/s) |
|---|---|---|---|
| 10 | $7.85398 \times 10^{-5}$ | $5 \times 10^{-6}$ m³/s | $6.37 \times 10^{-2}$ |
| 9 | $6.36173 \times 10^{-5}$ | $5 \times 10^{-6}$ m³/s | $7.86 \times 10^{-2}$ |
| 8 | $5.02655 \times 10^{-5}$ | $5 \times 10^{-6}$ m³/s | $9.95 \times 10^{-2}$ |
| 7 | $3.84845 \times 10^{-5}$ | $5 \times 10^{-6}$ m³/s | $1.30 \times 10^{-1}$ |
| 6 | $2.82743 \times 10^{-5}$ | $5 \times 10^{-6}$ m³/s | $1.77 \times 10^{-1}$ |
| 5 | $1.9635 \times 10^{-5}$ | $5 \times 10^{-6}$ m³/s | $2.55 \times 10^{-1}$ |
| 4 | $1.25664 \times 10^{-5}$ | $5 \times 10^{-6}$ m³/s | $3.98 \times 10^{-1}$ |
| 3.5 | $9.62113 \times 10^{-6}$ | $5 \times 10^{-6}$ m³/s | $5.20 \times 10^{-1}$ |
| 3 | $7.06858 \times 10^{-6}$ | $5 \times 10^{-6}$ m³/s | $7.07 \times 10^{-1}$ |
| 2.5 | $4.90874 \times 10^{-6}$ | $5 \times 10^{-6}$ m³/s | 1.02 |
| 2 | $3.14159 \times 10^{-6}$ | $5 \times 10^{-6}$ m³/s | 1.59 |
| 1.5 | $1.76715 \times 10^{-6}$ | $5 \times 10^{-6}$ m³/s | 2.83 |
| 1 | $7.85398 \times 10^{-7}$ | $5 \times 10^{-6}$ m³/s | 6.37 |
| 0.5 | $1.9635 \times 10^{-7}$ | $5 \times 10^{-6}$ m³/s | 25.5 |

Then, the behaviour of the fluid inside the sampling conduit was determined I (see Table 2) by calculating the Reynolds number (Re), in cases where Re is lower than 200 the flow is laminar, otherwise, the flow would be turbulent. Re was calculated by the following formula:

$$Re = \frac{V_m . D}{v_c}$$

Wherein $V_m$ is average speed, D is the internal diameter of the sampling probe and $v_c$ is the cinematic viscosity.

| D (mm) | d (kg/m$^3$) | V$_m$ (m/s) | v$_c$ (Pa.s) | Re |
|---|---|---|---|---|
| 10 | 1.145 | 6.37x10$^{-2}$ | 1.66x10$^{-5}$ | 44.04 |
| 9 | 1.145 | 7.86x10$^{-2}$ | 1.66x10$^{-5}$ | 48.94 |
| 8 | 1.145 | 9.95x10$^{-2}$ | 1.66x10$^{-5}$ | 55.06 |
| 7 | 1.145 | 1.30x10$^{-1}$ | 1.66x10$^{-5}$ | 62.92 |
| 6 | 1.145 | 1.77x10$^{-1}$ | 1.66x10$^{-5}$ | 73.41 |
| 5 | 1.145 | 2.55x10$^{-1}$ | 1.66x10$^{-5}$ | 88.09 |
| 4 | 1.145 | 3.98x10$^{-1}$ | 1.66x10$^{-5}$ | 110.11 |
| 3.5 | 1.145 | 5.20x10$^{-1}$ | 1.66x10$^{-5}$ | 125.84 |
| 3 | 1.145 | 7.07x10$^{-1}$ | 1.66x10$^{-5}$ | 146.81 |
| 2.5 | 1.145 | 1.02 | 1.66x10$^{-5}$ | 176.18 |
| 2 | 1.145 | 1.59 | 1.66x10$^{-5}$ | 220.22 |
| 1.5 | 1.145 | 2.83 | 1.66x10$^{-5}$ | 293.63 |
| 1 | 1.145 | 6.37 | 1.66x10$^{-5}$ | 440.44 |
| 0.5 | 1.145 | 25.5 | 1.66x10$^{-5}$ | 880.88 |

Table 2. Calculation of Reynolds values for different diameters.

The experiment showed (see table 2) that sampling conduits having an internal diameter equal to or greater than 0.5 mm have a Reynolds number (Re) lower than 2000 which indicates that inside the conduit the flow is laminar i.e. distributed in parallel layers.

Once the laminar behaviour was established, the instantaneous speed (V$_i$) in laminar flow was calculated by the following formula:

$$V_i = v.\left[1 - \left(\frac{r}{R}\right)^2\right]$$

Wherein v is the maximum speed, r is the distance to the center of the sampling conduit and R is the maximum radio of the sampling probe.

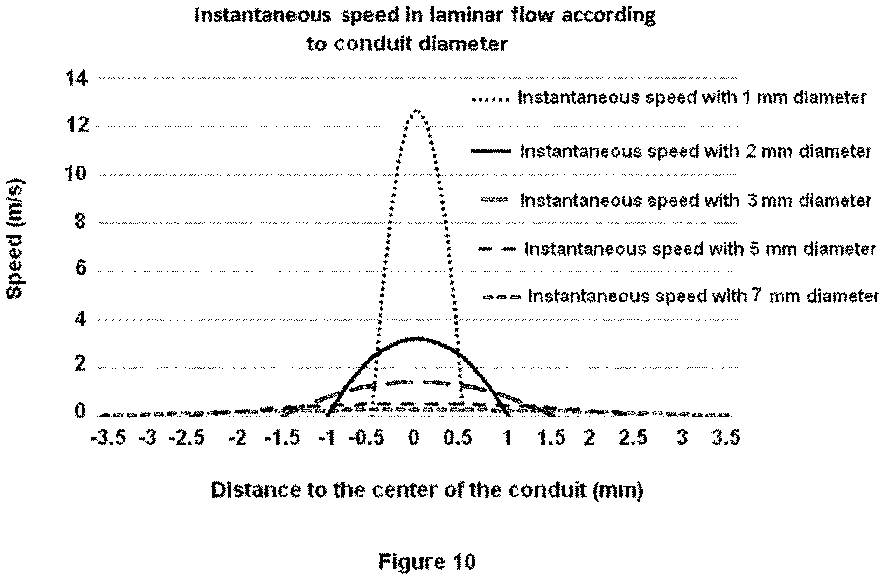

As seen in Figure 10, where different instantaneous speeds are shown, in cases of laminar flow, the maximum speed is located at the center of the conduit and, for sampling conduits having a lower diameter, the fluid speed near the inner walls of the sampling conduit would be higher than for those sampling conduits having a greater internal diameter. Consequently, with lower diameters a fluid film that may be created in the inner wall of the sampling conduit may be avoided or at least reduced, and therefore, the adhesion of propofol particles to the inner walls of the conduit may be prevented or at least reduced. In addition, the sampling system would show a faster response i.e. the propofol particles entering into and exiting from the sampling probe would correspond to those particles of the exhalation.

According to the present invention, a sampling probe such as probe 130 of Figure 1 comprises a heating element (see Figure 2) to heat at least a portion of the sampling conduit 133 and optionally, a sensor 160 e.g. a temperature sensor. By heating a portion or the whole sampling conduit, the undesired adhesion of particles to the inner surface of the sampling probe 130 may be substantially reduced. Such heating element may be e.g. a resistor, a heated fluid, etc.

According to the present invention, a resistor is used as heating element and it is coiled around the sampling conduit (see Figure 2). According to comparative examples, said resistor may form any other suitable shape e.g. parallel strips.

Figure 2 depicts a sampling probe 230 according to the present invention which comprises a resistance 210 coiled around the sampling conduit 233. According to some examples, the number of coils per length may vary along the sampling probe 230 i.e. to obtain different temperatures at different regions of the sampling probe 230. At least part of the sampling probe 230, i.e. the distal end, is intended to be placed inside the patient and thus, a maximum temperature should not be exceeded in order to avoid the risk of burning patient tissues. According to the present invention, the maximum temperature of the portion of the sampling probe 230 to be placed within the patient may be between 37° and 50° C.

In other examples, the sampling probe could be a resistive element e.g. a conductive polymer.

Additionally, the air exiting from the ventilator is usually refrigerated, and consequently, the heat released by the resistor 210 may be controlled, e.g. by regulating the current, to compensate the cool air flowing into the patient.

In some examples (see Figure 1) a temperature sensor may be provided to measure the temperature of the sampling probe. In other examples separate sensors, e.g. temperature sensors, may be placed in different portions of the sampling probe for more specific measurement. Consequently, the temperature of the heating element along different portions of the sampling conduit may be controlled so as to avoid an excessive temperature which may lead to damages in the oropharyngeal cavity.

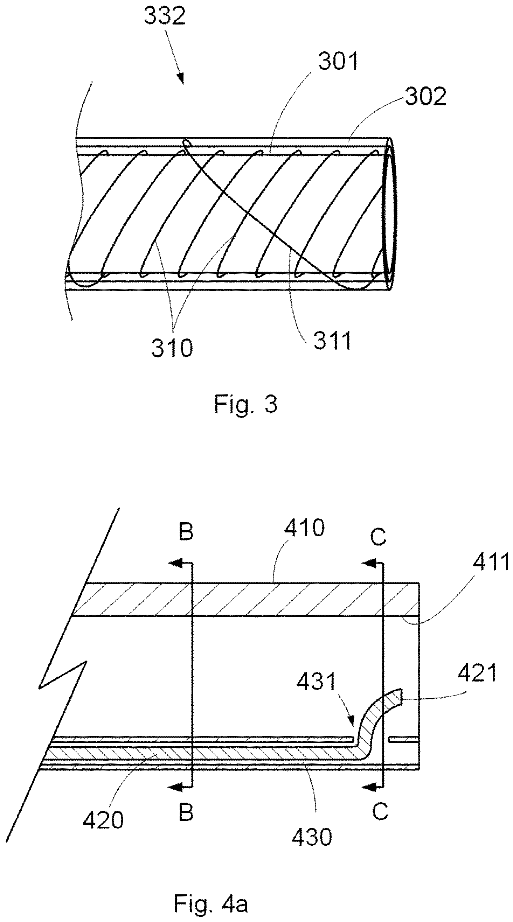

For security reasons, at least a layer of an electrically and/or thermally insulating material may be provided. Figure 3 shows a side view of an example of the distal end 332 of the sampling probe comprising two insulating layers 301, 302. Figure 3 depicts a first insulating layer 301 that at least partially covers the coils 310 wound from the power supply (not shown) towards the distal end 332 of the sampling probe. Figure 3 also shows a second insulating layer 302 which that at least partially cover the coils 311 that go backwards from the distal end 332 to the power supply (not shown). In this example, the insulating material is a medical grade insulating material. The diameter of the sampling probe depicted in the example of Figure 3 comprising two insulating layers and a coiled heating element is about 3 mm or less.

Figure 4a shows a lateral view of an endotracheal tube 410 having a hollow passageway 430 in its wall in which the sampling probe 420 may be located. The sampling tube 420 may comprise the features of any of the examples disclosed. In the example of Figure 4a, the hollow passageway 430 comprises a hole 431 at or substantially near the distal end of the endotracheal tube 410 from which at least a distal portion of the sampling probe 420, near the distal end 421, may protrude. In this example, the distal portion of the sampling probe 420 is curved so as to keep the distal end 421 with the inlet to the sampling conduit away from the inner walls 411 of the endotracheal tube. In other examples the distal portion of the sampling probe may comprise a different configuration e.g. a straight or angled slope.

The distal portion may be made of a relatively rigid material so as to maintain it separated from the inner wall 411 and prevent contamination e.g. prevent the water vapour condensed on the inner walls and/or corporal fluids such us mucus, from entering into the sampling conduit. In other examples the distal end may be centered, i.e. may be away from the inner wall of the endotracheal tube, by any suitable element e.g. a separating piece as disclosed later on.

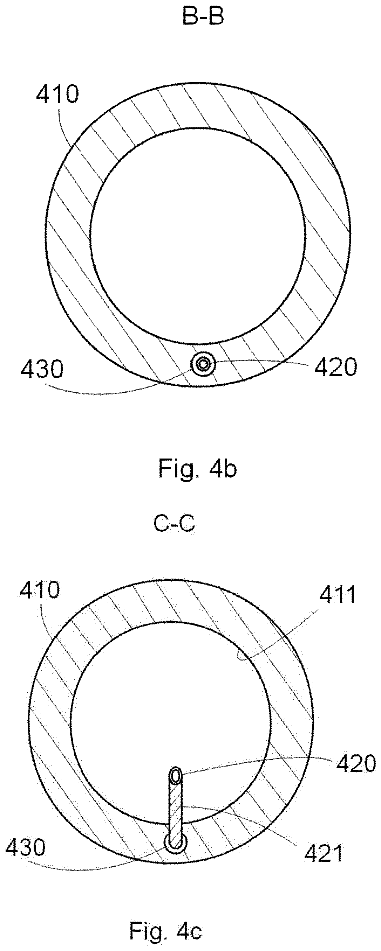

Figure 4b depicts a cross-section of the endotracheal tube 410 taken along line B-B of Figure 4a. Figure 4b shows the endotracheal tube 410 comprising the hollow passageway 430 in which the sampling probe 420 is placed.

Figure 4c shows a cross-section of the endotracheal tube 410 taken along line C-C of Figure 4a. Figure 4c shows the hollow passageway 430 from which the distal end 421 of the sampling probe 420 protrudes.

According to other examples, the endotracheal tube may comprise a rib (not shown) projecting substantially radially from its inner wall, in which the sampling probe may be located, such that the rib maintains the sampling probe, and therefore the sampling conduit, at a distance from the inner wall of the endotracheal tube. In such examples the distal end of the sampling probe may reach the end of the rib, and may also project from it. The rib itself may extend as far as the distal end of the endotracheal tube, or it may be slightly shorter, to prevent the distal end of the sampling probe from projecting with respect to the distal end of the endotracheal tube.

In alternative examples, the sampling probe may be attached e.g. by adhesive, to the inner wall of the endotracheal tube. In further examples, the sampling probe may be freely located inside the air passageway of the endotracheal tube e.g. without being fixed to the inner wall of the endotracheal tube. In such examples the distal end of the sampling conduit may also be maintained separate from the inner wall of the endotracheal tube.

The distal end of the sampling probe according to any of the examples herein disclosed may be located close to the distal end of the endotracheal tube, in order to withdraw the sample of exhaled air before some of the particles adhere to the inner wall of the endotracheal tube, thus improving the purity of the sampled air.

The distal end of the sampling probe may be slightly withdrawn inside the endotracheal tube, as shown in Figure 4a, or flush with it (see Figure 1), or in some cases projecting by a very small distance, provided that the shape of the end of the endotracheal tube and the position of the distal end of the sampling probe prevent contact of the sampling probe with the tissue of the oropharyngeal cavity.

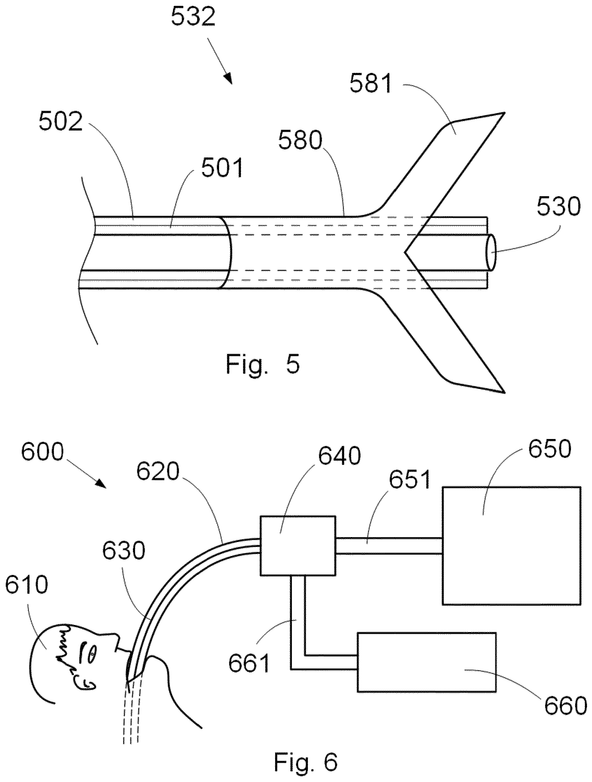

Figure 5 shows an example of a sampling probe 530 comprising two insulating layers 501, 502 which is provided with a separating piece 580 that is located at a distal end 532 thereof. Such separating piece 580 is adapted to keep at least the distal end 532 of the sampling probe 530 separated i.e. away from inner the walls of the endotracheal tube (not shown in Figure 5). By separating at least the distal end 532 of the sampling probe 530, the contact with the endotracheal tube and/or the oropharyngeal cavity is avoided, and thus, the contamination of the sampled air by e.g. vapour condensed on the endotracheal tube wall, a mucosa or any corporal fluid such as mucus, may be impeded or at least reduced. In other examples, a separating piece may be provided in additional or alternative positions other than the distal end of the sampling probe 530.

The separating piece 580 shown in Figure 5 comprises a plurality of flaps 581.

Other suitable elements may be employed, as long as they maintain at least the distal end 532 isolated from the inner wall of the endotracheal tube, for example centered. The flaps 581 in this example are made of an elastic and/or flexible material so as to facilitate the insertion and/or positioning of the sampling probe. The number of flaps 581 may vary.

Figure 6 depicts a system 600 according to the present invention, for analysing the composition of the air exhaled by a patient, which comprises a sampling device for sampling the exhaled air according to any of the examples hereinbefore disclosed, which in turn comprises an endotracheal tube 620 to be placed within the oropharyngeal cavity of a patient 610, a sampling probe 630 and a connection hub 640. The system also comprises a ventilator 650 and a fluid analyser 660 connected to the hub 640 through a breathing line 651 and a sampling line 661 respectively.

In some implementations the sampling probe 630, which has been disclosed above as extending between the patient and the connection hub, may also extend, with the same or similar features, to the sampling line 661 between the connection hub and the fluid analyser. Between the connection hub and the fluid analyser the sampling probe may be enclosed in a protective tube. The fluid analyser 660 may e.g. be a mass spectrometer, a sensor directed to measure specific components, e.g. compounds with low volatility, of the exhaled air, e.g. an anaesthetic drug, etc. In some examples, the fluid analyser may comprise a suction mechanism (not shown) to facilitate that the air exhaled from the patient reaches the fluid analyser. In an example, the suction mechanism may be inactive during inhalation phase and may be active in the exhalation phase. In other examples, the suction mechanism may be continuously in operation.

The system 600 may further comprise a flow control mechanism, e.g. a valve, as disclosed later on, to prevent the inhalation phase air flow from reaching the fluid analyser i.e. to avoid measuring compounds which are not part of the exhaled air. Such flow control mechanism may be controlled either by the fluid analyser or by the ventilator.

The ventilator may be any of the suitable commercially available devices.

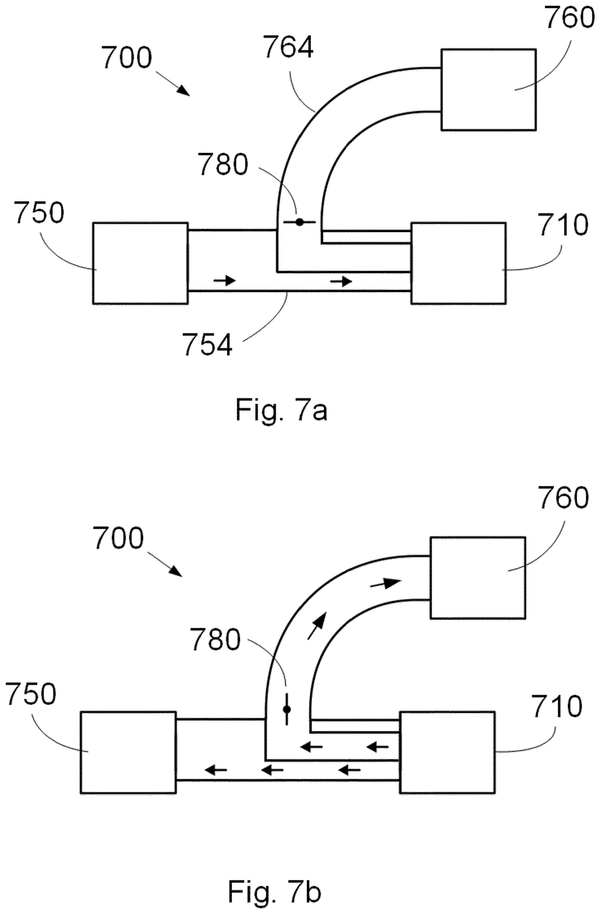

Figure 7a shows in more detail an example of part of a system 700 for analysing the exhaled air of a patient, such as system 600 of Figure 6. The system 700 comprises a breathing line 754 which connects an intubated patient 710 to a ventilator 750 and may comprise an endotracheal tube according to any of the examples disclosed. Within the breathing line 754 there may be a sampling line 764, for example a sampling probe according to any of the examples herein disclosed. The sampling line 764 may be configured to allow air to enter the fluid analyser 760 only during the exhalation phase and prevent an air flow from the ventilator from entering the analyser during an inhalation phase. In some examples the sampling line 764 may include all the features of the sampling probe hereinbefore disclosed.

For example, the sampling line of the example of Figure 7a comprises a valve 780 suitable to prevent inhaled air (see the arrows) from reaching the fluid analyser 760. According to this example, the valve 780 is controlled by the ventilator 750. When air is pumped towards the patient 710 the valve 780 is closed (as shown in Figure 7a) and when the air is exhaled from the patient towards the ventilator, i.e. the exhalation phase, the valve 780 is opened (see Figure 7b). In the example of Figures 7a and 7b a pivoting mechanism is provided to open/close the valve 780. According to some examples a pressure sensor may be provided to control the valve 780. According to other examples the valve may be controlled in other manners, for example by the fluid analyser.

Figure 7b depicts the system 700 shown in the example of Figure 7a but having the valve 780 open to let exhaled air reach the fluid analyser 760 (as shown by arrows).

The valve has been shown in the drawings arranged in the sampling line, i.e. the sampling probe, but according to other examples said valve may be placed in the connection hub or in the fluid analyser.

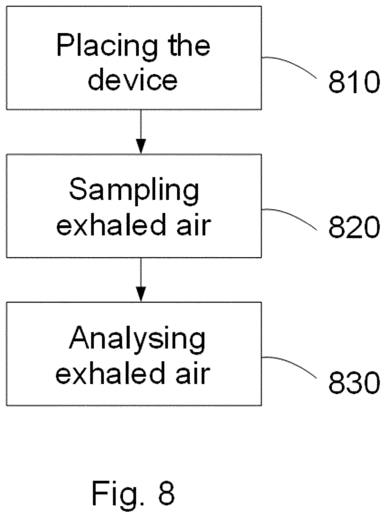

In Figure 8, an example of a method for analysing the composition of the exhaled air is shown, said method not being part of the claimed invention. Firstly, in block 810 an endotracheal tube of a sampling device according to any of the disclosed examples may be placed into a patient oropharyngeal cavity and an inflatable cuff may be inflated to retain the device in the correct position. According to some examples, a sampling probe placed within the endotracheal tube may be heated. During the exhalation, part of the exhaled air may be sampled in block 820. A valve such as disclosed above in relation with Figures 7a and 7b may be opened during exhalation and a sample of the exhaled air may be conveyed through the sampling probe to a fluid analyser, e.g. a mass spectrometer. The sampled exhaled air may then be analysed in the fluid analyser in block 830.

During subsequent inhalation induced by the ventilator, the valve may be closed so as to prevent air from entering the fluid analyser in this phase, such that only exhaled air is sampled and analysed, while air that is conveyed by the ventilator towards the patient, the composition of which is not relevant to assess the patient's response to the administered medicaments, does not reach the fluid analyser.

Although only a number of examples have been disclosed herein, other alternatives, modifications, uses and/or equivalents thereof are possible. Furthermore, all possible combinations of the described examples are also covered. Thus, the scope of the present disclosure should not be limited by particular examples, but should be determined only by a fair reading of the claims that follow. If reference signs related to drawings are placed in parentheses in a claim, they are solely for attempting to increase the intelligibility of the claim, and shall not be construed as limiting the scope of the claim.

**Claims**

1.  A device for sampling exhaled air of a patient, the device comprising:

    an endotracheal tube (120, 410) comprising a ventilation passageway, a proximal end to be connected to a connection hub (140) and a distal end to be placed inside a patient oropharyngeal cavity;
    a sampling probe (130, 230, 420) for sampling exhaled air of a patient, said sampling probe comprising a sampling conduit (133, 233) and being mounted within the endotracheal tube, wherein a proximal end of the sampling probe is adapted to be connected to a fluid analyser (660) and a distal end is adapted to receive samples of exhaled air, such that the sampling probe is adapted to convey the samples to the fluid analyser, and wherein such distal end is placed at the distal end of the endotracheal tube (120, 410),
    wherein the distal end of the sampling probe (130, 230, 420) is arranged separate and away from the inner wall of the endotracheal tube (120, 410) and wherein an internal diameter of the sampling conduit (133, 233) is equal to or lower than 3 mm,
    **characterized in that** it further comprises a heating element to heat at least a portion of the sampling conduit, the heating element comprising a resistor (210, 310, 311) coiled around the sampling conduit (133, 233),
    wherein at least part of the heated portion of the sampling probe is configured to be placed inside the patient, wherein the maximum temperature of the portion of the sampling probe to be placed within the patient is between 37°C and 50°C.

2.  The device according to claim 1, wherein the internal diameter of the sampling conduit (133, 233) is between 0.5 and 3 mm, preferably between 1 and 2 mm.

3.  The device according to claim 1 or 2, wherein the distal end of the sampling conduit (133, 233) is separated from the inner wall of the endotracheal tube (120, 410) by a separating piece (580) attached to the sampling probe (130, 230, 420).

4.  The device according to claim 3, wherein the separating piece (580) comprises flaps (581).

5.  The device according to any of claims 1 - 4 wherein at least part of the sampling probe (130, 230, 420) is arranged adjacent to the wall of the endotracheal tube (120, 410).

6.  The device according to claim 1, wherein the number of coils per length varies along the sampling conduit (133, 233).

7.  The device according to any of claims 1 - 6, wherein the sampling probe (130, 230, 420) comprises at least an external insulating layer (301, 302) covering the heating element.

8.  The device according to any of claims 1 - 7, wherein the sampling conduit (133, 233) is made of a material with low chemical reactivity to a compound to be analysed.

9.  The device according to any of claims 1 - 8, wherein the sampling conduit (133, 233) is made of a material having a coefficient of friction equal to or lower than 0.35.

10. The device according to any of claims 1 - 9, wherein the sampling conduit (133, 233) is made of a material selected among PEEK, modified PEEK, PET, PTFE and PPS.

11. The device according to any of claims 1 - 10, further comprising at least a temperature sensor (160) positioned to measure the temperature of at least one portion of the sampling probe (130, 230, 420).

12. A system for analyzing the composition of exhaled air, the system comprising:

a device according to any of claims 1 - 11;
a fluid analyser (660); and
a ventilator (650).

**Patentansprüche**

1. Eine Vorrichtung zur Probenahme von ausgeatmeter Luft eines Patienten, wobei die Vorrichtung Folgendes umfasst:

   einen Endotrachealtubus (120, 410) umfassend einen Beatmungsdurchgang, ein proximales Ende, das mit einem Verbindungsstück (140) zu verbinden ist, und ein distales Ende, das in einer Oropharynxhöhle eines Patienten zu platzieren ist;
   eine Probenahmesonde (130, 230, 420) zur Probenahme von ausgeatmeter Luft eines Patienten, wobei die Probenahmesonde eine Probenahmeleitung (133, 233) umfasst und innerhalb des Endotrachealtubus angebracht ist, wobei ein proximales Ende der Probenahmesonde angepasst ist, um mit einem Flüssigkeitsanalysator (660) verbunden zu werden und ein distales Ende angepasst ist, um Proben von ausgeatmeter Luft zu empfangen, so dass die Probenahmesonde angepasst ist, um die Proben zum Flüssigkeitsanalysator zu befördern, und wobei ein solches distales Ende am distalen Ende des Endotrachealtubus (120, 410) platziert ist,
   wobei das distale Ende der Probenahmesonde (130, 230, 420) getrennt und beabstandet von der Innenwand des Endotrachealtubus (120, 410) angeordnet ist und wobei ein Innendurchmesser der Probenahmeleitung (133, 233) gleich oder kleiner als 3 mm ist,
   **dadurch gekennzeichnet, dass** sie ferner ein Heizelement umfasst, um mindestens einen Abschnitt der Probenahmeleitung zu erwärmen, wobei das Heizelement ein Widerstand (210, 310, 311) umfasst, der um die Probenahmeleitung (133, 233) gewickelt ist,
   wobei mindestens ein Teil des erwärmten Abschnitts der Probenahmesonde konfiguriert ist, um innerhalb des Patienten platziert zu werden, wobei die maximale Temperatur des Teils der innerhalb des Patienten zu platzieren Probenahmesonde bei zwischen 37 °C und 50 °C liegt.

2. Die Vorrichtung nach Anspruch 1, wobei der Innendurchmesser der Probenahmeleitung (133, 233) zwischen 0,5 und 3 mm, vorzugsweise zwischen 1 und 2 mm beträgt.

3. Die Vorrichtung nach Anspruch 1 oder 2, wobei das distale Ende der Probenahmeleitung (133, 233) von der Innenwand des Endotrachealtubus (120, 410) durch ein an der Probenahmesonde (130, 230, 420) angebrachtes Trennstück (580) getrennt ist.

4. Die Vorrichtung nach Anspruch 3, wobei das Trennstück (580) Klappen (581) umfasst.

5. Die Vorrichtung nach einem der Ansprüche 1 bis 4, wobei mindestens ein Teil der Probenahmesonde (130, 230, 420) angrenzend an die Wand des Endotrachealtubus (120, 410) angeordnet ist.

6. Die Vorrichtung nach Anspruch 1, wobei die Anzahl der Spulen pro Länge entlang der Probenahmeleitung (133, 233) variiert.

7. Die Vorrichtung nach einem der Ansprüche 1 bis 6, wobei die Probenahmesonde (130, 230, 420) mindestens eine äußere Isolierschicht (301, 302) umfasst, die das Heizelement bedeckt.

8. Die Vorrichtung nach einem der Ansprüche 1 bis 7, wobei die Probenahmeleitung (133, 233) aus einem Material mit geringer chemischer Reaktivität gegenüber einer zu analysierenden Verbindung besteht.

9. Die Vorrichtung nach einem der Ansprüche 1 bis 8, wobei die Probenahmeleitung (133, 233) aus einem Material mit einem Reibungskoeffizienten gleich oder kleiner als 0,35 besteht.

10. Die Vorrichtung nach einem der Ansprüche 1 bis 9, wobei die Probenahmeleitung (133, 233) aus einem Material besteht, das aus PEEK, modifiziertem PEEK, PET, PTFE und PPS ausgewählt ist.

11. Die Vorrichtung nach einem der Ansprüche 1 bis 10, ferner umfassend mindestens einen Temperatursensor (160), der so positioniert ist, dass er die Temperatur von mindestens einem Teil der Probenahmesonde (130, 230, 420) misst.

**12.** Ein System zur Analyse der Zusammensetzung von ausgeatmeter Luft, wobei das System Folgendes umfasst:

eine Vorrichtung nach einem der Ansprüche 1 - 11;
einen Flüssigkeitsanalysator (660); und
ein Beatmungsgerät (650).

## Revendications

**1.** Un dispositif pour le prélèvement d'air expiré d'un patient, le dispositif comprenant :

un tube endotrachéal (120, 410) comprenant un passage de ventilation, une extrémité proximale à connecter à un raccord de connexion (140) et une extrémité distale à placer à l'intérieur d'une cavité oropharyngée d'un patient ;
une sonde de prélèvement (130, 230, 420) pour prélever de l'air expiré d'un patient, ladite sonde de prélèvement comprenant un conduit de prélèvement (133, 233) et étant montée à l'intérieur du tube endotrachéal, dans lequel une extrémité proximale de la sonde de prélèvement est adaptée pour être connectée à un analyseur de fluide (660) et une extrémité distale est adaptée pour recevoir des échantillons d'air expiré, de telle sorte que la sonde de prélèvement soit adaptée pour transporter les échantillons vers l'analyseur de fluide, et dans lequel telle extrémité distale est placée au niveau de l'extrémité distale du tube endotrachéal (120, 410), dans lequel l'extrémité distale de la sonde de prélèvement (130, 230, 420) est disposée séparément et à une certaine distance de la paroi interne du tube endotrachéal (120, 410) et dans lequel un diamètre interne du conduit de prélèvement (133, 233) est égal ou inférieur à 3 mm, **caractérisé en ce qu'**il comprend en outre un élément chauffant pour chauffer au moins une partie du conduit de prélèvement, l'élément chauffant comprenant une résistance (210, 310, 311) enroulée autour du conduit de prélèvement (133, 233), dans lequel au moins une partie de la partie chauffée de la sonde de prélèvement est configurée pour être placée dans l'intérieur du patient, dans lequel la température maximale de la partie de la sonde de prélèvement à être placée dans l'intérieur du patient est d'entre 37 °C et 50 °C.

**2.** Le dispositif selon la revendication 1, dans lequel le diamètre interne du conduit de prélèvement (133, 233) est d'entre 0,5 et 3 mm, de préférence d'entre 1 et 2 mm.

**3.** Le dispositif selon la revendication 1 ou 2, dans lequel l'extrémité distale du conduit de prélèvement (133, 233) est séparée de la paroi interne du tube endotrachéal (120, 410) par une pièce de séparation (580) fixée à la sonde de prélèvement (130, 230, 420).

**4.** Le dispositif selon la revendication 3, dans lequel la pièce de séparation (580) comprend des volets (581).

**5.** Le dispositif selon l'une quelconque des revendications 1 à 4, dans lequel au moins une partie de la sonde de prélèvement (130, 230, 420) est disposée adjacente à la paroi du tube endotrachéal (120, 410).

**6.** Le dispositif selon la revendication 1, dans lequel le nombre de bobines par longueur varie le long du conduit de prélèvement (133, 233).

**7.** Le dispositif selon l'une quelconque des revendications 1 à 6, dans lequel la sonde de prélèvement (130, 230, 420) comprend au moins une couche isolante externe (301, 302) recouvrant l'élément chauffant.

**8.** Le dispositif selon l'une quelconque des revendications 1 à 7, dans lequel le conduit de prélèvement (133, 233) est constitué d'un matériau à faible réactivité chimique vis-à-vis d'un composé à analyser.

**9.** Le dispositif selon l'une quelconque des revendications 1 à 8, dans lequel le conduit de prélèvement (133, 233) est constitué d'un matériau ayant un coefficient de frottement égal ou inférieur à 0,35.

**10.** Le dispositif selon l'une quelconque des revendications 1 à 9, dans lequel le conduit de prélèvement (133, 233) est constitué d'un matériau choisi parmi le PEEK, le PEEK modifié, le PET, le PTFE et le PPS.

**11.** Le dispositif selon l'une quelconque des revendications 1 à 10, comprenant en outre au moins un capteur de température (160) positionné pour mesurer la température d'au moins une partie de la sonde de prélèvement (130, 230, 420).

12. Un système d'analyse de la composition de l'air expiré, le système comprenant :

un dispositif selon l'une quelconque des revendications 1 à 11 ;
un analyseur de fluides (660) ; et
un respirateur artificiel (650).

Fig. 1

Fig. 2

332

301

302

310   311

Fig. 3

B   410   C

411

431   421

420   B   430   C

Fig. 4a

B-B

410

430          420

Fig. 4b

C-C

410                                    411

420

430                                   421

Fig. 4c

15

532

502

501

580

581

530

Fig. 5

600

620

630

610

640

651

650

661

660

Fig. 6

Fig. 7a

Fig. 7b

Fig. 8

Measured propofol (ppb) for different internal diameters of a sampling conduit

Figure 9

EP 3 548 129 B1

Figure 10

EP 3 548 129 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 16382589 A
- GB 2033759 A